# EUROPÄISCHE PATENTANMELDUNG

(11) **EP 4 474 815 A1**
(43) Veröffentlichungstag der Anmeldung: **11.12.2024**
(21) Anmeldenummer: 24176177.4
(22) Anmeldetag: 16.05.2024
(51) Int. Cl.: G01N 33/00

(54) **VERFAHREN ZUM BESTIMMEN EINER KORRELATIONSFUNKTION, VERFAHREN ZUR GASBESTIMMUNG UND GASSENSOR**

(30) Priorität: 05.06.2023 DE 102023205210
(71) Anmelder: Robert Bosch GmbH, 70442 Stuttgart (DE)
(72) Erfinder: Nolte, Philipp, 70839 Gerlingen (DE); Thaden, Eike Martin, 22527 Hamburg (DE); Mahesh Kumar, P Nallasivam, 641035 Coimbatore District Tamilnadu (IN); Chen, Wenwen, 71120 Grafenau (DE)

(57) **Zusammenfassung**

Die Erfindung betrifft ein Verfahren zum Bestimmen einer Korrelationsfunktion für die Verwendung mit einem Gassensor, umfassend: Bereitstellen (300) von Testmesswerten (302), die mittels eines Gassensors von einer oder mehreren verschiedenen Prüfsubstanzen in einer oder mehreren verschiedenen bestimmten Gasatmosphären erfasst worden sind; Bereitstellen (310) von Referenzmesswerten (312), die mittels eines Referenz-Gassensors von der einen oder den mehreren verschiedenen Prüfsubstanzen in der einen oder den mehreren verschiedenen Gasatmosphären erfasst worden sind; Bestimmen (320) der Korrelationsfunktion (322) aus den Testmesswerten und den Referenzmesswerten, wobei die Korrelationsfunktion Messwerte des Gassensors auf Messwerte des Referenz-Gassensors abbildet; und Bereitstellen (330) der Korrelationsfunktion zur Verwendung mit dem Gassensor und/oder einem Maschinenlernmodell, das auf den oder einen anderen Referenz-Gassensor trainiert ist.

## Beschreibung

Die vorliegende Erfindung betrifft ein Verfahren zum Bestimmen einer Korrelationsfunktion für die Verwendung mit einem Gassensor, ein Verfahren zur Gasbestimmung, eine Recheneinheit und ein Computerprogramm zu deren Durchführung, sowie einen Gassensor, insbesondere einen Multigassensor.

### Hintergrund der Erfindung

Gassensoren können verwendet werden, um Informationen über Gase wie z.B. deren Zusammensetzung oder deren Konzentration, insbesondere bei Spurengasen und/oder Gerüchen in Luft als Trägergas, zu bestimmen. Gassensoren, die verschiedene Gase (oder Gasgemische) verarbeiten können, werden auch als Multigassensoren bezeichnet. Hierbei kann auch von sog. elektronischen Nasen gesprochen werden.

### Offenbarung der Erfindung

Erfindungsgemäß werden ein Verfahren zum Bestimmen einer Korrelationsfunktion, ein Verfahren zur Gasbestimmung, ein Gassensor sowie eine Recheneinheit und ein Computerprogramm zur Durchführung der Verfahren mit den Merkmalen der unabhängigen Patentansprüche vorgeschlagen. Vorteilhafte Ausgestaltungen sind Gegenstand der Unteransprüche sowie der nachfolgenden Beschreibung.

Die Erfindung beschäftigt sich mit Gassensoren, insbesondere Multigassensoren. Unter Verwendung eines Gassensors können Bestandteile einer Gasatmosphäre qualitativ oder quantitativ bestimmt werden. Eine Gasatmosphäre umfasst eine Mischung von Gasen und/oder Dämpfen. Es kann sich auch überwiegend um Luft handeln, in der sich zusätzlich zu natürlichen Bestandteilen weitere Bestandteile befinden können. Diese einzelnen Bestandteile können z.B. im Sinne ihrer chemischen Stoffe (Elemente, Verbindungen) und deren Konzentrationen beschrieben werden, beispielsweise (molare) Konzentrationen von Ethanol, Kohlenmonoxid, Nikotin oder bestimmte Kältemittel (z.B. als Leckage aus Kühlanlagen / Fahrzeug-Klimaanlagen). Auch vorhandene Luftfeuchtigkeit kann unter diesen Begriff der gasförmigen Bestandteile als chemische Verbindung fallen. Der Zweck des Gassensors kann damit insbesondere die Bestimmung einer Teilmenge dieser chemischen Substanzen und ihrer (molaren) Konzentrationen sein ("Zielbestandteile"). Kommt in einem Anwendungsfall ein typisches Trägergas oder Trägergasgemisch vor (z.B. Luft), ist der relevante Informationsanteil insbesondere in den zusätzlichen Substanzen zu sehen, die grundsätzliche Zusammensetzung des Trägergases (Luft) kann als an sich bekannt angenommen werden oder ist bekannt und insofern nicht weiter relevant.

Die Bestandteile der Gasatmosphäre können aber auch in einer zusammenfassenden Weise betrachtet werden, vor allem bei Gerüchen. Z.B. können bei Zigarettenqualm, Parfüms oder Lebensmitteln als Geruchsträger eine Vielfalt von chemischen Verbindungen vorhanden sein, die auch nicht zwingend in einem von Probe zu Probe reproduzierbar festen molaren Verhältnis stehen. Hier ist der Zweck des Gassensors insbesondere, den Geruchstyp aus einer Menge von Zielgerüchen zu identifizieren (z.B. kategorisch ja/nein; oder in einer semi-quantitativen Abstufung, z.B. auf einer normierten Skala von 0 bis 1 je nach Verdünnungsgrad des emittierten Geruchs aus dem Geruchsträger mit Luft). Auch mit dieser alternativen, zusammenfassenden Bedeutung (ohne ausdrückliche Benennung der einzelnen chemischen Stoffe) kann der Zweck des Gassensors die Bestimmung einer Teilmenge vorhandener Gerüche als "Ziel-Bestandteile" der Gasatmosphäre verstanden werden.

Gassensoren können also verwendet werden, um Informationen über eine oder mehrere gasförmige Bestandteile - nachfolgend auch als Zielbestandteile bezeichnet - einer Gasatmosphäre zu bestimmen, je nach Kontext insbesondere im Sinne von chemischen Stoffen und molaren Konzentrationen oder, wie insbesondere bei Gerüchen in Luft als Trägermedium, im Sinne einer Erkennung der typischen Geruchsquelle. Die Informationen können also bei Gerüchen auch quantitativ, z.B. als Grad einer Verdünnung bewertet werden. Hierzu können mittels des Gassensors einer oder mehrere Messwerte zu einer Gasatmosphäre erfasst werden, aus denen dann die Informationen zu dem Ziel-Bestandteil (oder mehreren Zielbestandteilen) bestimmt werden können. Der Gassensor muss nicht zwingend alle Bestandteile erfassen, die in einem Anwendungsfall Bestandteil der Gasatmosphären werden oder sein können. Die Gewinnung von Information kann sich auf Ziel-Bestandteile beschränken. Die sonstigen Bestandteile werden im Folgenden auch als Störsubstanzen oder Störbestandteile bezeichnet, unabhängig davon, ob es um konkret benennbare chemische Stoffe oder um die Zusammenfassung von potenziell vielen chemischen Substanzen aus einem Geruchsträger geht.

Für das Bestimmen der Informationen von Zielbestandteilen aus dem einen oder den mehreren Messwerten, also z.B. zum Klassifizieren und/oder Quantifizieren von Gerüchen, kann ein Maschinenlernalgorithmus bzw. ein Maschinenlernmodell verwendet werden. Ein Maschinenlernmodell kann z.B. ein künstliches neuronales Netz umfassen oder ein solches sein. Das Maschinenlernmodell erhält dann den einen oder die mehreren Messwerte als Eingabe und bestimmt die Informationen zu dem Gas, die das Maschinenlernmodell dann als Ausgabe ausgibt.

Damit aus den Messwerten eine möglichst genaue Information bestimmt werden kann, ist es möglich, dass ein Maschinenlernmodell trainiert wird. Hierzu können sog. Trainings- und Test-Daten nötig sein, die typischerweise eine sehr hohe Anzahl verschiedener Messwerte zu verschiedenen Gasatmosphären umfassen. Die verschiedenen Messwerte der Trainings- und Test-Daten sollten möglichst alle Zielbestandteile (einzeln oder als Überlagerung mehrerer Zielbestandteile) bzw. die hierfür zu bestimmenden Informationen abdecken.

Zusätzlich ist es sinnvoll, auch die Anwesenheit der Störsubstanzen abzuprüfen, z.B. einzeln, überlagert und dabei idealerweise in Überlagerung mit den Zielbestandteilen. Besonders sinnvoll ist es, als Störsubstanzen verschiedene Feuchtigkeitswerte abzuprüfen, da Luftfeuchtigkeit in Alltags-Anwendungen einer hohen Bandbreite unterliegen kann und der Feuchteanteil Einfluss auf die Rohsignalwerte des Gassensors haben kann.

Je nach Aufbau einer Messzelle des Gassensors und Anordnung des Gassensors in der Anwendung können weitere physikalische Variationen der Atmosphäre (insbesondere der Gesamtdruck des Gases, Temperatur oder Strömungsgeschwindigkeit) entsprechend der Variation im Anwendungsfall sinnvoll sein, wenn diese Variationen in der Messzelle Einfluss auf die Signalbildung haben. Die entsprechend durch weitere physikalische Parameter präziser charakterisierte Atmosphäre wird im Folgenden als bestimmte Atmosphäre bezeichnet.

Umgekehrt brauchen diese physikalischen Variationen nicht weiter betrachtet werden, wenn z.B. durch Temperaturregelung, Gestaltung einer Pumpgeometrie etc. innerhalb des Gassensors gewährleistet ist, dass externe Schwankungen am Ort der Signalentstehung kompensiert werden.

Bei sog. überwachtem Lernen bzw. Trainieren können neben den Messwerten selbst dann auch die hierfür geltenden Informationen nötig sein, damit der Maschinenlernalgorithmus bzw. dessen Gewichte entsprechend angepasst werden können, sodass für bestimmte Messwerte die richtigen Informationen zu der einen oder den mehreren Substanzen ausgegeben werden.

Das Trainieren eines solchen Maschinenlernalgorithmus bzw. Maschinenlernmodells funktioniert an sich sehr gut, je mehr Messwerte für die Trainingsdaten verfügbar sind, umso genauer kann die Gasbestimmung mittels des trainierten Maschinenlernmodells erfolgen.

Wie sich gezeigt hat, liefern verschiedene Gassensoren jedoch unterschiedliche Rohsignale, also Messwerte. Dies gilt auch für an sich bau- oder typengleiche Gassensoren (sog. Exemplarstreuung). Die direkte Nutzung eines trainierten Maschinenlernmodells unter Verwendung der abweichenden Daten würde nun zu einem Genauigkeitsverlust führen. Für die Aufrechterhaltung der Genauigkeit müsste ein Maschinenlernmodell für jeden individuellen Gassensor erstellt werden bzw. der Maschinenlernalgorithmus müsste nachtrainiert werden.

Eine umfangreiche Datenerhebung über Tage oder Wochen lässt sich in einer Massenproduktion allerdings nicht sinnvoll für jedes einzelne Gerät bzw. jeden einzelnen Gassensoren durchführen. Vor allem kann der Gassensor (bzw. die elektronische Nase) z.B. nur eine von mehreren Komponenten in einem technischen Gerät sein (z.B. bei einem Leckage-Suchgerät, einem Haushaltsgerät, bei einer Luftqualitätsmessung im Fahrzeuginnenraum, einer Lüftungsanlage, etc.) und der erforderliche Aufwand müsste in einem sinnvollen Verhältnis zum Anteil der Geruchsfunktion zur Gesamtfunktion des Gerätes stehen.

Vor diesem Hintergrund wird nun die Verwendung einer Korrelationsfunktion vorgeschlagen, mit der ein auf einem Referenz-Gassensor trainiertes Maschinenlernmodell mit einem anderen Gassensor verwendet werden kann, dabei aber trotzdem sehr genaue Informationen zu dem einen oder den mehreren Bestandteilen der Atmosphäre erhalten werden können.

Zum Bestimmen einer solchen Korrelationsfunktion werden Testmesswerte, die mittels eines Gassensors von einer oder mehreren verschiedenen Prüfsubstanzen in einer oder mehreren verschiedenen Gasatmosphären erfasst worden sind, bereitgestellt. Dabei können die eine oder die mehreren Prüfsubstanzen insbesondere auch in verschiedenen Mischungen der Sorten und/oder Konzentrationen vorliegen und Zielbestandteile und/oder Störsubstanzen beinhalten. Es können auch dritte Substanzgruppen als Prüfsubstanzen verwendet werden, die im Anwendungsfall weder Zielsubstanz noch Störsubstanz sind. Bei diesem Gassensor handelt es sich um denjenigen Gassensor, für den die Korrelationsfunktion später gelten soll.

Weiterhin werden Referenzmesswerte bereitstellt, die mittels eines Referenz-Gassensors von der einen oder den mehreren verschiedenen Prüfsubstanzen in der einen oder den mehreren verschiedenen Gasatmosphären erfasst worden sind. Auch hier können die eine oder die mehreren Prüfsubstanzen insbesondere auch in verschiedenen Mischungen der Sorten und/oder Konzentrationen vorliegen und Zielsubstanzen und/oder Störsubstanden oder Störgase beinhalten. Es können auch dritte Substanzgruppen als Prüfsubstanzen verwendet werden, die im Anwendungsfall weder Zielsubstanz noch Störsubstanz sind. Bei dem Referenz-Gassensor handelt es sich um denjenigen Gassensor, für den ein trainiertes Maschinenlernmodell vorliegt, oder aber zumindest einen Gassensor, von dem bekannt ist, dass ein trainiertes Maschinenlernmodell damit hinreichend genau funktioniert (z.B. kann der Referenz-Gassensor selbst auch eine Korrelationsfunktion nutzen). Der Gassensor und der Referenz-Gassensor sollten dabei aber von der gleichen Art bzw. vom gleichen Typ sein, es handelt sich dann lediglich um verschiedene Exemplare eines bestimmten Typs von Gassensor.

Grundsätzlich können die Testmesswerte und die Referenzmesswerte unabhängig voneinander erfasst werden, wobei dann beachtet werden sollte, dass für jede verwendete Gasatmosphäre mit den Prüfsubstanzen einander entsprechende Test- und Referenzmesswerte vorliegen.

Besonders zweckmäßig ist es insofern aber, z.B. eine Prüfanordnung zu verwenden, bei der zugleich die Test- und Referenzmessewerte für dann jeweils eine angelegte Gasatmosphäre bei bestimmten Prüfsubstanzen erfasst werden können. Denkbar ist hierbei auch, zugleich mehrere individuelle Gassensoren und einen Referenz-Gassensor zu verwenden, sodass zugleich die Testmesswerte für später mehrere Korrelationsfunktionen erfasst werden können.

Die Korrelationsfunktion wird dann aus den Testmesswerten und den Referenzmesswerten bestimmt, wobei die Korrelationsfunktion Messwerte des Gassensors auf Messwerte des Referenz-Gassensors abbildet. Dies bedeutet, dass ein bestimmter Messwert bei einer oder mehreren bestimmten Prüfsubstanzen in den bestimmten Gasatmosphären für einen Gassensor durch die Korrelationsfunktion (bzw. deren Anwendung) derart abgeändert wird, dass er einen Wert hat, als wäre er vom Referenz-Gassensor bei genau dieser einen oder mehreren bestimmten Prüfsubstanzen in der bestimmten Gasatmosphäre erfasst worden. Die Korrelationsfunktion wird dann zur Verwendung mit dem Gassensor und insbesondere einem Maschinenlernmodell, das auf den oder einen anderen Referenz-Gassensor trainiert ist, bereitgestellt.

In einer Ausführungsform sind die Referenzmesswerte, und insbesondere auch die Testmesswerte, für eines oder mehrere vorbestimmte Signalmerkale des Gassensors erfasst worden. Ein Gassensor bzw. Multigassensor kann z.B. mehrere reale und/oder virtuellen Einzelsensoren für Substanzen und vorteilhafterweise auch für Temperatur und/oder Feuchtegehalt und/oder Druck aufweisen, z.B. Chemiresistoren (ein einzelner oder mehrere Rezeptoren; virtuelle Sensoren z.B. durch thermische Betriebsweisen). Es kann sich auch um optische und/oder elektrochemische Sensoren oder eine Kombination verschiedener Sensortypen handeln. Dabei kommt es auch nicht darauf an, ob es sich bei den realen Einzelsensoren um baulich getrennte Sensorkomponenten oder integrierte Kombinationen (z.B. auf einem gemeinsamen Chip) handelt.

Der Gassensor erfasst bei einer Messung dann eines oder mehrere (allgemein n) verschiedene Merkmale (Signalmerkale) als Rohsignale entsprechend dem Sensortyp. So kann z.B. ein Signal an einem Widerstand an einer leitfähigen gasempfindlichen Schicht oder ein transformiertes Signal aus dem Rohsignal (z.B. Leitfähigkeit 1/R) erhalten werden. Bei anderen Sensortypen kann z.B. ein amperometrisches Signal, eine Lichtextinktion etc. erhalten werden. Ganz allgemein kann ein Signalmerkmal als eine physikalische Größe, z.B. Spannung, Strom etc. sein.

Es kann dann eine merkmalsindividuelle Korrelationsfunktion bestimmt werden, wobei alle vorhandenen merkmalsindividuellen Korrelationsfunktionen zusammen die Korrelationsfunktion für die Verendung mit dem Gassensor darstellen.

Weiterhin ist es dann bevorzugt, wenn die eine oder die mehreren verschiedenen Prüfsubstanzen und/oder die eine oder die mehreren verschiedenen bestimmten Gasatmosphären derart gewählt sind, dass die Referenzmesswerte eine vorgegebene Messwertspanne für das eine oder jedes der mehreren vorbestimmten Signalmerkmale abdecken.

Das individuelle Gerät, d.h. der Gassensor für den die Korrelationsfunktion zu bestimmen ist, wird, z.B. gemeinsam mit dem Referenz-Gassensor verschiedenen (z.B. einer Anzahl k) Gasatmosphären ausgesetzt. Die k Gasatmosphären sind dann so ausgewählt, dass sie am Referenz-Gassensor in jedem der n Signalmerkmale eine vorbestimmte Messwertspanne (von niedrig bis hoch, z.B. von 1V bis 4V wenn der gesamte mögliche Spannungsbereich von 0V bis 5V reicht) abdecken, wie sie auch im realen Anwendungsfall vorkommen kann.

Es müssen jedoch nicht vollumfänglich alle relevanten Gasatmosphären abgedeckt werden, also nicht alle potentiell möglichen, verschiedenen Substanzen mit Konzentrationsintervallen von niedrig bis hoch bzw. alle realen Geruchsproben; es kommt lediglich auf die Messwertspannen in den Signalmerkmalen an, da nur darüber die Messwerte variieren.

Damit ist die Anzahl von Gasatmosphären, die für die Bestimmung der Korrelationsfunktion nötig ist, gegenüber einer umfangreichen Datensammlung wie z.B. bei einem Nachtrainieren des Maschinenlernmodells erheblich erniedrigt. Die Auswahl und Festlegung dieser Gasatmosphären mit den Prüfsubstanzen sind vielfältig umsetzbar, beispielsweise können es mehrere gasförmige Substanzen sein, davon jede Substanz separat, jeweils in verschiedenen Konzentrationen. Es können Gemische aus mindestens zwei gasförmigen Substanzen sein. Es können reale Geruchssubstanzen (z.B. Lebensmittelprobe, Zigarettenqualm) in unterschiedlichen Verdünnungsgraden in Luft sein. Es können verschiedene Feuchtigkeitsstufen abgedeckt werden. Die Konzentrationen müssen nicht exakt bekannt sein und dürfen auch Ungenauigkeiten unterliegen. Etwaige Schwankungen und/oder Ungenauigkeiten werden nämlich bei hinreichend guter Korrelation gleichermaßen im Referenz-Gassensor und im Gassensor (Testgerät) in den Sensormerkmalen abgebildet.

Es kann eine Überprüfung der Korrelation zwischen den Signalmerkmalen im Referenz-Gassensor und im Gassensor erfolgen. Im trivialen Idealfall wären die Test- und Referenzmesswerte identisch. Die Bewertung kann mit gängigen Verfahren der Statistik erfolgen, z.B. Pearson-Koeffizient oder über einen Fit mit einer empirisch bewährten Kurvenform. Diese Kurve kann als Kalibrierkurve herangezogen werden. Die Quantifizierung von Abweichungen kann beispielsweise über quadratische Abweichungen der Messwerte von der angenommenen Kalibrierkurve erfolgen.

Zur Gasbestimmung, d.h. zur Bestimmung von Zielbestandteilen einer Gasatmosphäre, werden dann einer oder mehrere Messwerte bereitgestellt, die mittels eines Gassensors von einer oder mehreren Bestandteilen einer Gasatmosphäre erfasst worden sind. Aus dem einen oder den mehreren Messwerten des Gassensors werden dann mittels einer Korrelationsfunktion einer oder mehrere Vergleichsmesswerte bestimmt. Die Korrelationsfunktion ist dabei für den Gassensor und einen Referenz-Gassensor bestimmt worden. Dies kann wie vorstehend beschrieben erfolgt sein. Die Korrelationsfunktion kann also insbesondere auch eine Kombination mehrerer merkmalsindividueller Korrelationsfunktionen sein.

Aus dem einen oder den mehreren Vergleichsmesswerten werden dann Informationen zu dem einen oder den mehreren Zielbestandteilen bestimmt, und zwar mittels eines Maschinenlernmodells, das den einen oder die mehreren Vergleichsmesswerte als Eingabe erhält und die Information zu der einen oder den mehreren Zielbestandteilen als Ausgabe ausgibt. Das Maschinenlernmodell ist dabei vorzugsweise auf den Referenz-Gassensor, mit dem die Korrelationsfunktion bestimmt worden ist, trainiert. Denkbar ist aber auch, dass das Maschinenlernmodell auf einen anderen Referenz-Gassensor trainiert worden ist. Die Informationen zu der einen oder den mehreren Zielbestandteilen der Gasatmosphäre werden dann bereitgestellt.

Das Bereitstellen der Informationen (also das Ergebnis der Messung bzw. der Gasbestimmung) kann z.B. auf einem Display, in einen Speicher oder zur Weiterverarbeitung zu anderen Zwecken in dem Gerät, in dem der Gassensor eingebaut ist (z. B. Leckage-Suchgerät, Haushaltsgerät, Luftqualitätsmessung im Fahrzeuginnenraum, Lüftungsanlage), erfolgen.

Eine Betriebssoftware des individuellen Gassensors (Testobjekt) dient an sich zum Erfassen aller Messwerte an diesem Gassensor bzw. Gerät. Das Gerät bzw. der Gassensor oder eine Recheneinheit (z.B. Steuereinheit) des Gassensors weist dasselbe trainierte Maschinenlernmodell wie der Referenzsensor bzw. dessen Recheneinheit auf, d.h. es erfolgt eine Inferenz auf Basis der Signalwerte. Die Betriebssoftware weist z.B. eine Schnittstelle auf, die die tatsächlichen Messwerte auf Basis der Korrelationsfunktion in die Vergleichswerte umrechnet (d.h. eine Emulation der Messwerte des Referenzgeräts auf Basis der Messwerte im individuellen Gerät.

Das trainierte Maschinenlernmodell kann aber auch außerhalb des Gassensors bzw. dessen Recheneinheit realisiert sein, beispielsweise auf einem Server (z.B. in einer sog. Cloud); es kann dann eine Cloud-basierte Abfrage auf Basis der Messwerte erfolgen. Hier sind wiederum verschiedene Varianten möglich und bevorzugt. Die Umrechnung zwischen den Messwerten und dem Vergleichsmesswerte mittels der Korrelationsfunktion kann lokal auf dem Gassensor bzw. dessen Recheneinheit erfolgen. Die Vergleichsmesswerte können dann an die Cloud bzw. den Server übergeben werden. Die Umrechnung kann aber auch auf dem Server bzw. in der Cloud erfolgen. Hierfür können die Messwerte an den Server bzw. die Cloud übergeben werden. Die Korrelationsfunktion oder Parameter, die die Korrelationsfunktion eindeutig bestimmen, sollten dann passend zu jedem individuellen Gassensor auf dem Server bzw. in der Cloud hinterlegt sein und eindeutig abgerufen werden können, z.B. über eine Geräte-ID.

Der Referenz-Gassensor (bzw. das Referenzgerät), mit dem die Korrelationsfunktion bestimmt wird, kann dasselbe Gerät (d.h. derselbe Referenz-Gassensor) sein, an dem die Trainingsdaten für das Training des Maschinenlernmodells aufgezeichnet wurden. Der Referenz-Gassensor kann aber ein anderes Individuum als das Gerät sein, auf dem trainiert wurde, aber vorzugsweise nur dann, falls die Funktion und Messgenauigkeit dieses Gerätes nachgewiesen worden sind. Ein mit dem vorgestellten Verfahren angepasster Gassensor (Test-Objekt) kann somit selbst zu einem neuen Referenz-Gassensor bzw. Referenzgerät in einer späteren Messung werden, sofern die volle Funktion in einem (umfangreicheren) Test nachgewiesen wurde, d.h. alle als relevant erachteten Metriken mit dem kalibrierten Maschinenlernmodell auf diesem Gerät erreichen mindestens eine definierte Güte.

Für den Gassensor (Testobjekt bzw. individuelles Gerät) kann es je nach Realisierungsvariante bestimmte Anforderungen geben. Die Korrelationsfunktion (oder auch Kalibrierfunktion oder Kalibrierparameter) sollten z.B. in einem elektronischen Speicher hinterlegt werden können, auf den eine ausführende Recheneinheit oder Steuereinheit unmittelbaren Zugriff hat. Die Sensoren und der Speicher der Kalibrierparameter können gemeinsam in ein austauschbares Modul ausgelagert werden. Wenn die Korrelations- bzw. Kalibrierfunktion (bzw. die merkmalsindividuellen Korrelationsfunktionen) auf einem Server bzw. in einer Cloud hinterlegt ist und nicht lokal auf dem Gerät, sollte durch eine Geräte-ID sichergestellt werden, dass passend zum individuellen Gerät der entsprechende Satz von Kalibrierparametern aufgerufen werden kann.

Zum Bestimmen der Korrelationsfunktion kann z.B. eine Prüfeinrichtung verwendet werden. Eine Gasdosiereinheit kann z.B. zu einer Maschine in der Fertigung oder Prüfung erweitert werden, indem die Erstellung und Bewertung der Korrelationen zwischen den Sensormerkmalen von Gassensor und Referenz-Gassensor automatisiert erfolgen und nach erfolgreicher Prüfung die Übertragung der Korrelationsfunktion bzw. der Kalibirierparameter in einen Speicher der elektronischen Nasen oder in einen Cloud-Speicher ermöglichen. Beispielhafte Kriterien für die Selektion (innerhalb/außerhalb einer definierten Toleranz) sind z.B. Korrelationskoeffizienten zwischen den n Sätzen zu den Test- und Referenzmesswerten, eine Güte der Fits zwischen den Testmesswerten und der Korrelationsfunktion /oder deren individuellen n Kalibrierfunktionen, d.h. für jedes Signalmerkmal), oder Ausreisserpunkte gegenüber der Korrelationsfunktion.

Damit zu jedem Zeitpunkt der Kalibriermessung die gleiche Gasatmosphäre in den Testobjekten und im Referenzgerät gewährleistet ist, kann z.B. ein symmetrischer Aufbau aller Gasentnahmestellen aller Geräte (Referenz, Testobjekte) aus einer Gase-Mischstation vorteilhaft sein. Dann könnten selbst dann Messwerte verwendet werden, wenn sich die Gasatmosphäre noch nicht auf einen finalen, stabilen Endwert eingestellt hat. Beispiele sind ein diametraler Einbau der beiden Geräte oder ein sternförmiger Einbau von mehreren Geräten an ein Kammervolumen. Der Einlass der Gase in das Kammervolumen kann über eine Pumpe oder einen Massenflussregler erfolgen. Der Einlass der Gase kann von jeder elektronischen Nase über eine eingebaute Pumpe erfolgen. Der Einlass der Gase in jede elektronische Nase kann auch ohne eingebaute Pumpen durch den Überdruck im Kammervolumen erfolgen. Verfügen die elektronischen Nasen über Pumpen, so kann der Einlass vorteilhafterweise mit einem Überschuss an Gasfluss erfolgen, der über einen Auslass an dem Kammervolumen abgelassen wird.

Eine erfindungsgemäße Recheneinheit, z.B. eine Steuereinheit eines Gassensors oder ein Computer, ist, insbesondere programmtechnisch, dazu eingerichtet, ein erfindungsgemäßes Verfahren durchzuführen.

Auch die Implementierung eines erfindungsgemäßen Verfahrens in Form eines Computerprogramms oder Computerprogrammprodukts mit Programmcode zur Durchführung aller Verfahrensschritte ist vorteilhaft, da dies besonders geringe Kosten verursacht, insbesondere wenn ein ausführendes Steuergerät noch für weitere Aufgaben genutzt wird und daher ohnehin vorhanden ist. Schließlich ist ein maschinenlesbares Speichermedium vorgesehen mit einem darauf gespeicherten Computerprogramm wie oben beschrieben. Geeignete Speichermedien bzw. Datenträger zur Bereitstellung des Computerprogramms sind insbesondere magnetische, optische und elektrische Speicher, wie z.B. Festplatten, Flash-Speicher, EEPROMs, DVDs u.a.m. Auch ein Download eines Programms über Computernetze (Internet, Intranet usw.) ist möglich. Ein solcher Download kann dabei drahtgebunden bzw. kabelgebunden oder drahtlos (z.B. über ein WLAN-Netz, eine 3G-, 4G-, 5G- oder 6G-Verbindung, etc.) erfolgen.

Weitere Vorteile und Ausgestaltungen der Erfindung ergeben sich aus der Beschreibung und der beiliegenden Zeichnung.

Die Erfindung ist anhand von Ausführungsbeispielen in der Zeichnung schematisch dargestellt und wird im Folgenden unter Bezugnahme auf die Zeichnung beschrieben.

### Kurze Beschreibung der Zeichnungen

- Figur 1: zeigt schematisch einen erfindungsgemäßen Gassensor in einer bevorzugten Ausführungsform.
- Figuren 2a und 2b: zeigen schematisch Anordnungen zur Durchführung erfindungsgemäßer Verfahren in bevorzugten Ausführungsformen.
- Figur 3: zeigt schematisch einen Ablauf eines erfindungsgemäßen Verfahrens in einer bevorzugten Ausführungsform.
- Figur 4: zeigt schematisch Diagramme zur Erläuterung der Erfindung.
- Figur 5: zeigt schematisch einen Ablauf eines erfindungsgemäßen Verfahrens in einer weiteren bevorzugten Ausführungsform.

### Ausführungsform(en) der Erfindung

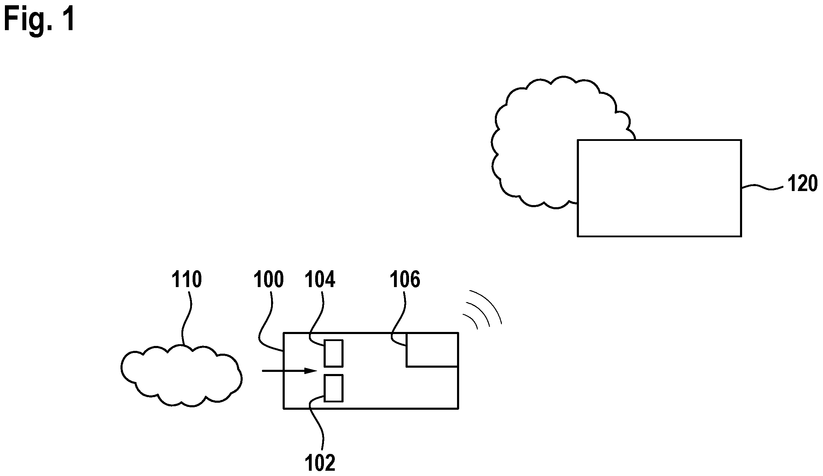

In Figur 1 ist schematisch ein erfindungsgemäßer Gassensor 100 in einer bevorzugten Ausführungsform dargestellt. Beispielhaft weist der Gassensor 100 zwei Sensorelemente 102, 104 auf, es handelt sich also um einen Multigassensor. Weiterhin weist der Gassensor 100 eine Rechen- oder Steuereinheit 106 auf, auf der z.B. eine Betriebssoftware laufen kann, um Messwerte zu erfassen und ggf. zu verarbeiten. Beispielhaft ist die Steuereinheit 106 auch zur drahtlosen Kommunikation eingerichtet, um z.B. Daten an einen übergeordneten Server 120 (z.B. in einer sog. Cloud) zu senden und/oder Daten von dort zu empfangen.

Weiterhin ist beispielhaft eine Gasatmosphäre 110 angedeutet, deren Zielbestandteile mittels des Gassensors 100 bestimmt werden können bzw. zu der unter Verwendung des Gassensors 100 Informationen bestimmt werden können. Ein möglicher Ablauf, Informationen zu den Zielbestandteilen in 110 zu bestimmen, soll später näher erläutert werden.

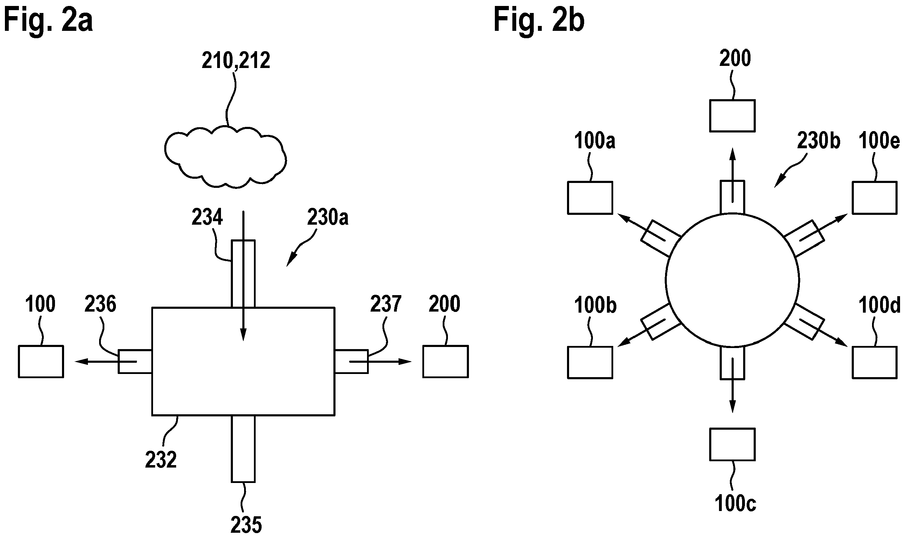

In den Figuren 2a und 2b sind schematisch Anordnungen zur Durchführung erfindungsgemäßer Verfahren in bevorzugten Ausführungsformen dargestellt, und zwar, zum Bestimmen einer Korrelationsfunktion bzw. mehrerer merkmalsindividueller Korrelationsfunktionen. In Figur 2a ist eine Prüfanordnung 230a grob schematisch gezeigt. Die Prüfanordnung 230a weist eine Kammer oder ein Volumen 232 auf, einen Einlass 234 und einen Auslass 235. Außerdem weist die Prüfanordnung 230a zwei Anschlüsse 236, 237 auf.

An dem Anschluss 236 ist beispielhaft ein Gassensor 100 (z.B. derjenige aus Figur 1) angeschlossen; es handelt sich um das sog. Testobjekt oder Testgerät. An dem Anschluss 237 ist beispielhaft ein Referenz-Gassensor 200 angeschlossen; es handelt sich um das sog. Referenzgerät.

Durch den Einlass 234 kann nun eine Gasatmosphäre 212 mit einem oder mehreren Bestandteilen 210 in die Kammer 232 eingeleitet werden, die dann durch den Auslass 235 wieder aus der Kammer 232 herausgeführt wird. Die Anschlüsse 236, 237 sind hierbei z.B. derart ausgebildet, dass die Gasatmosphäre mit den Bestandteilen gleichermaßen in den Gassensor 100 sowie den Referenz-Gassensor 200 gelangt. Insbesondere sollten z.B. geometrische Abmessungen wie z.B. Entfernungen derart sein, dass im Gassensor 100 und im Referenz-Gassensor 200 die Gasatmosphäre zu jedem Zeitpunkt möglichst mit gleicher Konzentration und gleicher Zusammensetzung erfasst werden.

Die Gasatmosphäre mit der Substanz kann in das Volumen 232 über z.B. über Pumpen angesaugt werden oder über Massenflussregler eingelassen werden. Die Gasatmosphäre mit der Substanz kann über jeweils eigene Pumpen in den elektronischen Nasen (d.h. Gassensor 100 und Referenz-Gassensor 200) oder durch Überdruck in der Kammer 232 zu den einzelnen elektronischen Nasen gelangen.

Auf diese Weise können verschiedene Gasatmosphären mit verschiedenen Prüfsubstanzen als gasförmigen Bestandteile verwendet werden. Dabei sollen unter verschiedenen Prüfsubstanzen verschiedene individuelle Substanzen, insbesondere aber auch verschiedene Mischungen verschiedener Substanzen (einschließlich Gasen) verstanden werden. Sie können die Ziel- und typische Störsubstanzen gemäß der Anwendung der elektronischen Nase beinhalten. Sie können aber auch sonstige ausgewählte Substanzen sein (unabhängig davon, ob sie in der Anwendung als Ziel- oder Störbestandteil der Gasatmosphäre vorkommen), die helfen, die beabsichtigten Messwertspannen aller Signalmerkmale abzudecken. Unter verschiedenen bestimmten Gasatmosphären können dagegen zusätzlich zur stofflichen Zusammensetzung verschiedene weitere physikalische Bedingungen wie Drücke, Temperaturen, Strömungsgeschwindigkeiten verstanden werden.

Auf diese Weise können Testmesswerte für den Gassensor 100 und Referenzmesswerte für den Referenz-Gassensor 200 bestimmt werden.

In Figur 2b ist eine Prüfanordnung 230b grob schematisch gezeigt. Die Prüfanordnung 230b kann grundsätzlich vergleichbar zur Prüfanordnung 230a aufgebaut sein, jedoch sind hier beispielhaft fünf verschiedene Gassensoren 100a, 100b, 100c, 100d, 100e und ein Referenz-Gassensor 200 gezeigt. Jeder der Gassensoren 100a, 100b, 100c, 100d, 100e kann hier wie der Gassensor 100 in Figur 2a verwendet werden, wobei es sich um verschiedene Exemplare handelt, für die jeweils eine Gassensor-individuelle Korrelationsfunktion (wieder ggf. mit jeweils mehreren merkmalsindividuellen Korrelationsfunktionen) bestimmt werden kann. Einlass und Auslass für die Gasatmosphären sind hier nicht dargestellt.

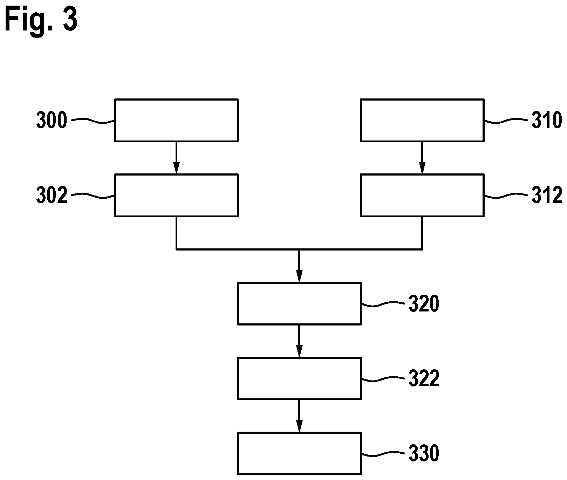

In Figur 3 ist schematisch ein Ablauf eines erfindungsgemäßen Verfahrens in einer bevorzugten Ausführungsform dargestellt. Es handelt sich um ein Verfahren zum Bestimmen einer Korrelationsfunktion; hierzu kann z.B. die Prüfanordnung gemäß Figur 2a verwendet werden.

In einem Schritt 300 werden Testmesswerte 302 bereitgestellt, die mittels eines Gassensors wie z.B. dem Gassensor 100 von einem oder mehreren verschiedenen Prüfsubstanzen in einer oder mehreren verschiedenen bestimmten Gasatmosphären erfasst worden sind. In einem Schritt 310 werden Referenzmesswerte 312 bereitgestellt, die mittels eines Referenz-Gassensors wie z.B. dem Referenz-Gassensor 200 von dem einen oder den mehreren verschiedenen Prüfsubstanzen in der einen oder den mehreren verschiedenen bestimmten Gasatmosphären erfasst worden sind.

In einem Schritt 320 wird dann eine Korrelationsfunktion 322 aus den Testmesswerten und den Referenzmesswerten bestimmt, wobei die Korrelationsfunktion Messwerte des Gassensors auf Messwerte des Referenz-Gassensors abbildet. In einem Schritt 330 wird die Korrelationsfunktion dann zur Verwendung mit dem Gassensor und insbesondere einem Maschinenlernmodell, das auf den oder einen anderen Referenz-Gassensor trainiert ist, bereitgestellt.

Vorzugsweise sind die Referenzmesswerte 312, und insbesondere auch die Testmesswerte 302, für eines oder mehrere vorbestimmte Signalmerkale des Gassensors erfasst worden. Bevorzugt sind die eine oder die mehreren verschiedenen Prüfsubstanzen und/oder die eine oder die mehreren verschiedenen bestimmten Gasatmosphären derart gewählt, dass die Referenzmesswerte 312 eine vorgegebene Messwertspanne für das eine oder jedes der mehreren vorbestimmten Signalmerkmale abdecken. Es werden dann merkmalsindividuellen Korrelationsfunktionen bestimmt, die zusammen die Korrelationsfunktion ergeben.

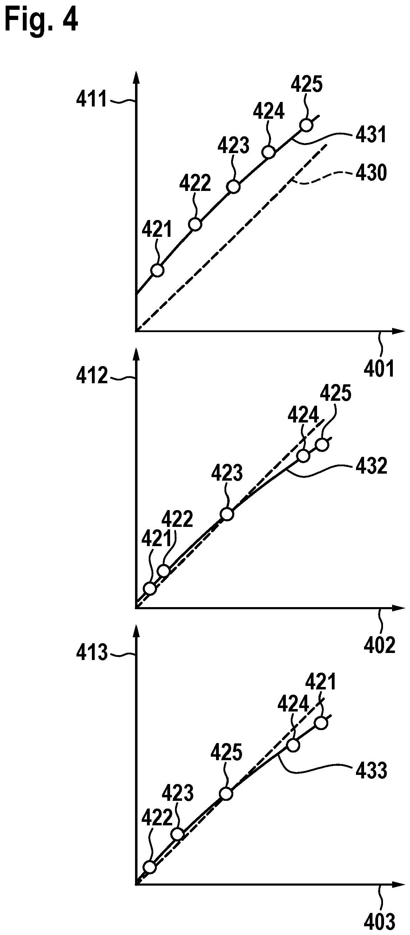

In Figur 4 sind hierzu schematisch Diagramme zur Erläuterung der Erfindung dargestellt. Die Diagramme zeigen Messwerte für drei verschiede Signalmerkmale 401, 402, 403 eines Gassensors (Testgerät) und zugehörige Referenzmesswerte für diese drei Signalmerkmale 411, 412, 413 eines Referenz-Gassensors (Referenzgerät). Dabei sind jeweils fünf verschiedene Gasatmosphären 421, 422, 423, 424, 425 mit jeweils z.B. verschiedenen Prüfsubstanzen verwendet worden.

In einem trivialen Idealfall würden die Messwerte und Referenzmesswerte bei derselben Gasatmosphäre identisch sein; dies ist z.B. im oberen Diagramm mit der Linie 430 angedeutet. Im praktischen Fall, bei dem der Gassensor und der Referenz-Gassensor zwar vom selben Typ sind, jedoch unterschiedliche Exemplare sind, weichen die Messwerte und Referenzmesswerte voneinander ab. Die Korrelation zwischen Messwerten und Referenzmesswerten sind mit den Punkten für die verschiedenen Gasatmosphären 421, 422, 423, 424, 425 dargestellt.

Für den Fall des Signalmerkmals 401 bzw. 411 im oberen Diagramm ist bei der Gasatmosphäre 421 der Messwert (Achse 401) sehr niedrig, der zugehörige Referenzmesswert (Achse 411) hingegen deutlich größer. An diesem Beispiel sind deutlich mögliche Abweichungen von einer Idealkurve (401=411) zu sehen, z.B. durch Offset, Steigung, Linienkrümmung.

Im mittleren Diagramm mit den Signalmerkmalen 402 bzw. 412 soll im Wesentlichen veranschaulicht werden, dass sich für dieselben Prüfgase (also Substanzen in einer Gasatmosphäre), die bezüglich des oberen Diagramms (bzw. des Signalmerkmals 401 bzw. 411) zumindest in etwa äquidistant verteilt sind, bezüglich eines anderen Signalmerkmals - hier eben 402 bzw. 412 - die äquidistante Verteilung ändern kann.

Im unteren Diagramm ist eine andere Reihenfolge zu der Gasatmosphären zu sehen, und zwar bezogen auf die jeweiligen Werte bzw. Signalstärke des Signalmerkmals 403 bzw. 413. So ein Fall könnte z.B. auftreten, wenn Gasatmosphären durch gemischte Zudosierung aus zwei Substanzen A, B in Luft verwendet werden (z.B. Mischung 1: "viel A, wenig B", Mischung 2: "viel B, wenig A") und das Sensormerkmal 402 bzw. 412 stark auf Substanz A und schwach auf Substanz B reagiert, Sensormerkmal 403 bzw. 413 hingegen stark auf Substanz B und schwach auf Substanz A reagiert. Die Reihenfolge und Punktdichte der Mess- bzw. Referenzmesswerte bezüglich jedes Prüfgases kann im Allgemeinen also für jedes Signalmerkmal verschieden sein.

Auf diese Weise lässt sich für das Signalmerkmal 401 bzw. 411 eine Korrelation 431 - und damit eine merkmalsindividuelle Korrelationsfunktion - bestimmen. Gleichermaßen kann für die Signalmerkmale 402 bzw. 412 sowie 403 bzw. 413 jeweils eine Korrelation 432, 433 bestimmt werden. Die Korrelationen 431, 432, 433 bzw. die entsprechenden merkmalsindividuellen Korrelationsfunktionen können dann zusammen die Korrelationsfunktion bilden.

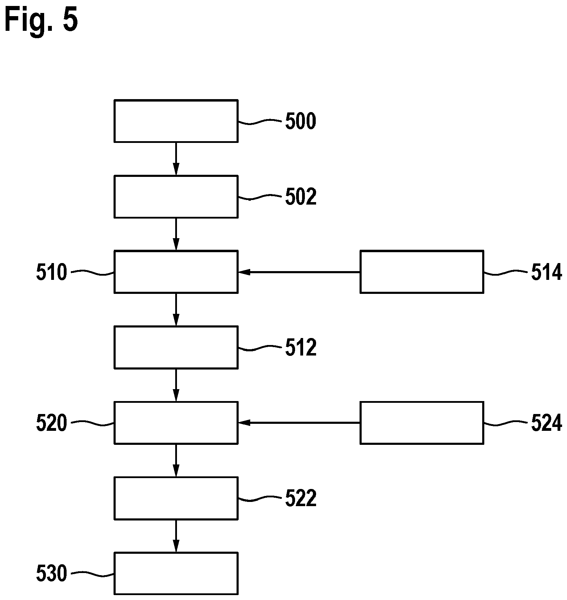

In Figur 5 ist schematisch ein Ablauf eines erfindungsgemäßen Verfahrens in einer weiteren bevorzugten Ausführungsform dargestellt. Es handelt sich um ein Verfahren zur Bestimmung von Zielbestandteilen einer Gasatmosphäre (Gasbestimmung) bzw. zum Bestimmen einer Information hierzu; hierzu kann z.B. die Anordnung gemäß Figur 1 verwendet werden.

In einem Schritt 500 werden einer oder mehrere Messwerte 502 bereitgestellt, die mittels eines Gassensors von einem oder mehreren Bestandteilen einer Gasatmosphäre erfasst worden sind. Dies kann das Messen selbst umfassen.

In einem Schritt 510 werden, aus dem einen oder den mehreren Messwerten des Gassensors, einer oder mehrere Vergleichsmesswerte 512 bestimmt, und zwar mittels einer Korrelationsfunktion 514; die Korrelationsfunktion (umfassend z.B. mehrere merkmalsindividuelle Korrelationsfunktionen) ist für den Gassensor und einen Referenz-Gassensor bestimmt worden, wie z.B. in Bezug auf Figur 3 erläutert.

In einem Schritt 520 werden aus dem einen oder den mehreren Vergleichsmesswerten Informationen 522 zu der einen oder den mehreren Substanzen bestimmt, und zwar mittels eines Maschinenlernmodells 524, das den einen oder die mehreren Vergleichsmesswerte als Eingabe erhält und die Information zu dem einen oder den mehreren Zielbestandteilen der Gasatmosphäre als Ausgabe ausgibt. In einem Schritt 530 werden die Informationen zu der einen oder den mehreren Substanzen bereitgestellt, also z.B. auf einem Display angezeigt.

Hierbei gibt es, wie schon erwähnt, verschiedene bevorzugte Möglichkeiten hinsichtlich des Ortes der Durchführung verschiedener Schritte. Das Messen bzw. Erfassen der Messwerte kann mittels des Gassensors erfolgen. Das Bestimmen der Vergleichswerte daraus, d.h. Schritt 510, kann z.B. auf dem Gassensor bzw. dessen Steuereinheit erfolgen, oder aber auch auf einem übergeordneten Server, z.B. in der Cloud. In letzterem Fall können die Messwerte von dem Gassensor zu dem Server übermittelt werden, wie in Bezug auf Figur 1 erwähnt.

Falls Schritt 510 auf dem Server erfolgt, kann auch Schritt 520 auf dem Server erfolgen, d.h. das Bestimmen der Informationen zu dem Gas. Die Informationen können dann wieder zurück auf den Gassensor bzw. dessen Steuereinheit übermittelt werden, damit diese dort z.B. angezeigt werden können. Ebenso können die Informationen aber auch (ggf. auch zusätzlich) auf dem Server bereitstellt und z.B. gespeichert werden.

Falls Schritt 510 auf dem Gassensor erfolgt, kann auch Schritt 520 auf dem Gassensor erfolgen. Eine Datenübermittlung ist dann nicht nötig. Ebenso kann aber Schritt 520 auf dem Server erfolgen. Dann können die Vergleichsmesswerte von dem Gassensor zu dem Server übermittelt werden, wie in Bezug auf Figur 1 erwähnt.

Denkbar ist an sich auch, dass nur Schritt 510 auf dem Server erfolgt, Schritt 520 hingegen wieder auf dem Gassensor bzw. dessen Steuereinheit.

## Patentansprüche

1. Verfahren zum Bestimmen einer Korrelationsfunktion für die Verwendung mit einem Gassensor (100), umfassend:
Bereitstellen (300) von Testmesswerten (302), die mittels eines Gassensors (100) von einer oder mehreren verschiedenen Prüfsubstanzen in einer oder mehreren verschiedenen bestimmten Gasatmosphären (421-425) erfasst worden sind;
Bereitstellen (310) von Referenzmesswerten (312), die mittels eines Referenz-Gassensors (200) von der einen oder den mehreren verschiedenen Prüfsubstanzen in der einen oder den mehreren verschiedenen Gasatmosphären (421 -425) erfasst worden sind;
Bestimmen (320) der Korrelationsfunktion (322) aus den Testmesswerten und den Referenzmesswerten, wobei die Korrelationsfunktion Messwerte des Gassensors auf Messwerte des Referenz-Gassensors abbildet; und
Bereitstellen (330) der Korrelationsfunktion zur Verwendung mit dem Gassensor und/oder einem Maschinenlernmodell, das auf den oder einen anderen Referenz-Gassensor trainiert ist.

2. Verfahren nach Anspruch 1, wobei die Referenzmesswerte (312), oder die Referenzmesswerte (312) und die Testmesswerte, für eines oder mehrere vorbestimmte Signalmerkale (401, 402, 403) des Gassensors erfasst worden sind.

3. Verfahren nach Anspruch 2, wobei die eine oder die mehreren verschiedenen Prüf-Substanzen und/oder die eine oder die mehreren verschiedenen bestimmten Gasatmosphären (421-425) derart gewählt sind, dass die Referenzmesswerte eine vorgegebene Messwertspanne für das eine oder jedes der mehreren vorbestimmten Signalmerkmale abdecken.

4. Verfahren zur Bestimmung von Zielbestandteilen einer Gasatmosphäre, umfassend:
Bereitstellen (500) eines oder mehrerer Messwerte (502), die mittels eines Gassensors (100) von einem oder mehrere Bestandteilen einer Gasatmosphäre erfasst worden sind;
Bestimmen (510), aus dem einen oder den mehreren Messwerten des Gassensors, eines oder mehrerer Vergleichsmesswerte (512) mittels einer Korrelationsfunktion (514), wobei die Korrelationsfunktion für den Gassensor (100) und einen Referenz-Gassensor (200) bestimmt worden ist;
Bestimmen (520), aus dem einen oder den mehreren Vergleichsmesswerten, von Informationen (522) zu dem einen oder den mehreren Zielbestandteilen der Gasatmosphäre mittels eines Maschinenlernmodells (524), das den einen oder die mehreren Vergleichsmesswerte als Eingabe erhält und die Information zu dem einen oder den mehreren Zielbestandteilen als Ausgabe ausgibt; und
Bereitstellen (530) der Informationen zu dem einen oder den mehreren Ziel-Bestandteilen.

5. Verfahren nach Anspruch 4, wobei das Maschinenlernmodell (524) auf den oder einen anderen Referenz-Gassensor (200) trainiert ist.

6. Verfahren nach Anspruch 4 oder 5, die Korrelationsfunktion gemäß einem Verfahren nach einem der Ansprüche 1 bis 3 bestimmt worden ist.

7. Verfahren nach einem der vorstehenden Ansprüche, wobei der Gassensor (100) als Multigassensor ausgebildet ist.

8. Recheneinheit (120), die dazu eingerichtet ist, alle Verfahrensschritte eines Verfahrens nach einem der Ansprüche 1 bis 3 oder nach Anspruch 7 in Rückbezug auf einen der Ansprüche 1 bis 3 durchzuführen.

9. Recheneinheit (106), die dazu eingerichtet ist, zumindest einen Verfahrensschritt eines Verfahrens nach einem der Ansprüche 4 bis 6 oder nach Anspruch 7 in Rückbezug auf einen der Ansprüche 4 bis 6 durchzuführen.

10. Gassensor (100) mit einer Recheneinheit (106) nach Anspruch 9.

11. Gassensor (100) nach Anspruch 10, der als Multigassensor ausgebildet ist.

12. Computerprogramm, das eine Recheneinheit dazu veranlasst, alle Verfahrensschritte eines Verfahrens nach einem der Ansprüche 1 bis 3 oder nach Anspruch 7 in Rückbezug auf einen der Ansprüche 1 bis 3 durchzuführen, oder zumindest einen Verfahrensschritt eines Verfahrens nach einem der Ansprüche 4 bis 6 oder nach Anspruch 7 in Rückbezug auf einen der Ansprüche 4 bis 6 durchzuführen, wenn es auf der Recheneinheit ausgeführt wird.

13. Maschinenlesbares Speichermedium mit einem darauf gespeicherten Computerprogramm nach Anspruch 12.
